# EUROPÄISCHE PATENTSCHRIFT

(11) **EP 3 545 836 B1**
(45) Veröffentlichungstag und Bekanntmachung des Hinweises auf die Patenterteilung: **17.06.2020**
(21) Anmeldenummer: 18164693.6
(22) Anmeldetag: 28.03.2018
(51) Int. Cl.: A61B 5/055, A61B 5/053, A61B 5/00, G01R 33/48

(54) **VERFAHREN ZUM DURCHFÜHREN EINER ELEKTRISCHE-IMPEDANZ-TOMOGRAPHIE MIT HILFE EINER MR-ANLAGE**
METHOD FOR CARRYING OUT ELECTRICAL IMPEDANCE TOMOGRAPHY WITH THE AID OF A MR INSTALLATION
PROCÉDÉ DE MISE EN UVRE D'UNE TOMOGRAPHIE PAR IMPÉDANCE ÉLECTRIQUE À L'AIDE D'UNE INSTALLATION MR

(43) Veröffentlichungstag der Anmeldung: 02.10.2019
(73) Patentinhaber: Siemens Healthcare GmbH, 91052 Erlangen (DE)
(72) Erfinder: Speier, Peter, 91056 Erlangen (DE)

(56) Entgegenhaltungen:
- DE-A1-102015 224 158
- US-A1- 2013 072 780
- US-A1- 2017 303 991
- US-B1- 6 397 095
- US-B2- 7 511 492

## Beschreibung

Die vorliegende Erfindung betrifft ein Verfahren zum Durchführen einer Elektrische-Impedanz-Tomographie mit Hilfe einer Magnetresonanz (MR)-Anlage. Weiter werden eine entsprechende MR-Anlage, ein Computerprogrammprodukt und ein elektronisch lesbarer Datenträger bereitgestellt.

Die Elektrische-Impedanz-Tomographie (EIT) ist ein nichtinvasives Bildgebungsverfahren, welches auf Messungen von elektrischen Leitfähigkeiten im menschlichen Körper basiert. Insbesondere beruht das Messverfahren darauf, dass sich die elektrischen Leitfähigkeiten biologischer Gewebe je nach Beschaffenheit (absolute EIT) und/oder funktionellem Zustand (funktionelle oder relative EIT) unterscheiden. Neben den Ansätzen der absoluten und funktionellen EIT, bei denen zumeist Wechselströme einer einzigen Frequenz genutzt werden, kann man auch Wechselströme verschiedener Wellenlängen einspeisen, wie beispielsweise in dem Wikipedia Artikel "Elektrische Impedanz-Tomografie, abgerufen am 27.02.2018 unter [https://de.wikipedia.org/wiki/Elektrische_Impedanz-Tomografie], bekannt ist.

Insbesondere können bei einer speziellen Technik der EIT mehrere Oberflächenelektroden an einem Objekt positioniert werden, wobei zwischen jeweils zwei Elektroden elektrische Wechselströme fließen, während simultan mit Hilfe der anderen Elektroden das elektrische Potential registriert wird. Die detektierten Oberflächenströme, bzw. das detektierte elektrische Potential, hängen von der Stromverteilung im Objekt ab, die durch die elektrische Impedanzverteilung der Objekte beeinflusst wird. Ziel ist es, eine ortsaufgelöste Impedanzverteilung zu berechnen, welche die gemessenen Oberflächenströme erzeugt. Dieses Inversionsproblem ist jedoch schlechtgestellt und kann nur mit Hilfe strenger Einschränkungen in Bezug auf die Geometrie und Struktur des Objekts gelöst werden.

In der Erdölförderung wird die EIT seit den 1930er Jahren eingesetzt und hat sich als grundlegende Informationsquelle für die kommerzielle In-Situ-Formationsbewertung etabliert. Die meisten geometrischen Grundmodelle weisen unendlich breite, elektrisch homogene Formationsschichten auf, die orthogonal zur Bohrung ausgerichtet sind. Ströme werden mit Hilfe von Elektroden, die die Formation berühren, oder bei höheren Frequenzen von Antennenanordnungen durch Induktion in die Formation eingespeist, wie beispielsweise aus der Druckschrift "Geophysical Well Logging" von Jay Tittman in Academic Press 1986, ISBN 0-12-691390-0, Kapitel 2.7 und 3.1, bekannt ist.

EIT-Verfahren werden auch bei medizinischen Problemen wie Perfusion und Mammographie angewandt, eine kommerzielle medizinische Anwendung ist dabei beispielsweise die ortsaufgelöste Darstellung bei der Lungenbeatmung eines Patienten mit Hilfe eines um den Oberkörper gelegten Sensorgürtels.

Aus der Druckschrift US 8929970 B2 ist ein Verfahren zur Detektion und Bewertung räumlich geordneter pathologischer Veränderungen bei einer medizinischen Untersuchungsperson durch Beobachtung von Phasenänderungen in MRI-Spin-Signalen bekannt, welche durch zeitlich variierende elektrische Potentialunterschiede in extern plazierten Faraday-Schilden hervorgerufen werden. Im Gegensatz zur EIT ist die räumliche Auflösung dieses Verfahrens diejenige der MRT-Messung. Ein Anwendungsbeispiel ist die Erkennung von Brustverletzungen. Die in der Druckschrift US 8929970 B2 beschriebene Methode unterscheidet sich von dem erfindungsgemäßen Verfahren dadurch, dass die in der US 8929970 B2 beschriebenen induzierten Ströme so hoch sind, dass sie lokale Magnetfelder erzeugen, welche stark genug sind, um meßbare Veränderungen in der lokalen Spin-Dynamik zu erzeugen. Diese Veränderungen in Folge analysiert, um lokale elektrische Eigenschaften abzuleiten.

Aus den Druckschriften US 7511492 B2 und US 7795870 B2 ist ein in eine MRT-Messung integriertes Hochfrequenz-Impedanz-Abbildungsverfahren (RF Impedance Mapping, RFIM), eine Variante der EIT, bekannt, welche eine induktive Kopplung anstelle einer galvanischen Kopplung bei der MR-Frequenz verwendet. Die Wechselwirkung der MR-Technik mit den induzierten Strömen wird genutzt, um die Modellinversion zu stabilisieren. B1-Mapping wird als eine mögliche Anwendung beschrieben. Der darin beschriebene Scanner kombiniert MR mit RFIM, indem er die MR-Empfangsspulen für die elektrische Messung verwendet, erforderlich sind jedoch zusätzliche Sende- und Empfangsfähigkeiten, d.h. zusätzliche Schaltungen, für alle lokalen Spulen, um deren Impedanz zu messen und zu überwachen. Weiter werden nur die HF-Spulen gemeinsam verwendet, jedoch nicht das HF-Erzeugungs- und Empfangssystem. Daher muss die MR-Messung adaptiert werden, so dass zwischen dem Senden von MR HF-Pulsen und Empfangen von MR-Signalen die RFIM-Messung eingeschoben werden kann, es müssen also EIT-Messungen mit den MR-Messungen zeitlich abwechselnd durchgeführt werden. Da eine MR-Messsequenz aus vielen Anregungen und Auslesen besteht spricht man dabei von einem Verweben der Ereignisse, im Sinne von ineinander verschachtelten Verfahren. Jedoch sind simultane, in anderen Worten zeitlich parallele, in dem Sinne von voneinander unabhängige, MR- und RFIM-Messungen dadurch nicht möglich, da die Spulen während der beiden Messungen an separate Elektronik angeschlossen sind.

Aus den Druckschriften DE 102015224162 A1, DE 102015224158 A1, DE 102015224162 B4, DE 102016215044 A1 sowie aus der Masterarbeit "Informationsgehalt eines neuartigen MR-Navigators in Bezug auf physiologische Vorgänge" von Lea Schröder, Friedrich-Alexander-Universität Erlangen-Nürnberg, 2015, ist ein Verfahren für die Bestimmung von Bewegungen, insbesondere von Atembewegungen sowie von Herzbewegungen mittels eines Pilot-Tone- (PT)-Signals bekannt. Das PT-Signal, welches eine HF-Frequenz umfasst, wird von einer PT-Sendespule kontinuierlich erzeugt und zusammen mit dem MR-Signal erfasst. Das gesendete Pilot-Tone-Signal erfährt durch das Untersuchungsobjekt eine Modulation, insbesondere eine Modulation der Phase und der Amplitude des PT-Signals. Da die Phase des Pilottons bekannt ist, lässt sich daraus der Zeitpunkt der Erfassung des MR-Signals ableiten.

Zusammenfassend werden im Stand der Technik für die Umsetzung von EIT-Messungen spezielle EIT-Vorrichtungen benötigt. Dazu gehören Signalgeneratoren, Signalapplikationshardware (Elektroden), Signalempfangshardware (Elektroden), Signalanalysehardware (Verstärker, Detektoren), Computer zur Signalverarbeitung und Modellinversion. Zusätzlich müssen Modellbeschränkungen generiert und in die Rekonstruktion eingespeist werden. Diese können aus allgemeinen anatomischen Modellen oder, besser gesagt, individuell optimiert, aus separaten Untersuchungen mit hochauflösenden bildgebenden Verfahren, wie beispielsweise CT, MR, oder vergleichbaren Untersuchungsmethoden stammen.

Es besteht daher Bedarf nach einem verbesserten Verfahren für Elektrische-Impedanz-Tomographie, welches mit geringerem Aufwand und mit kürzeren Untersuchungszeiten durchgeführt werden kann. Es besteht weiter Bedarf danach, die Qualität und die Effizienz der bestehenden EIT-Techniken zu verbessern, um bestehende EIT-Anwendungen zu stabilisieren und neue Anwendungen zu eröffnen.

Diese Aufgabe wird mit den Merkmalen der unabhängigen Ansprüche gelöst. In den abhängigen Ansprüchen sind weitere vorteilhafte Ausführungsformen der Erfindung beschrieben.

Gemäß einem ersten Aspekt wird ein Verfahren zum Durchführen einer Elektrische-Impedanz-Tomographie (EIT) bereitgestellt, welches mithilfe einer MR-Anlage umfassend eine Vielzahl von HF-Spulen durchgeführt wird. Die MR-Anlage kann eine MR-Anlage mit integrierter Pilot-Tone-Navigation sein.

In einem ersten Schritt wird ein HF-Puls mittels einer ersten Gruppe von HF-Spulen zum Anregen von Spins in dem Untersuchungsobjekt gesendet. In einem weiteren Schritt werden MR-Signale aus dem Untersuchungsobjekt basierend auf dem HF-Puls mittels einer zweiten Gruppe von HF-Spulen empfangen. In einem zusätzlichen Schritt werden kontinuierliche HF-Signale, mittels mindestens einer HF-Spule gesendet, welche Amplituden aufweisen, die in der Größenordnung der Amplituden der MR-Signale liegen.

In einem weiteren Schritt werden modulierte kontinuierliche HF-Signale aus dem Untersuchungsobjekt in Antwort auf die kontinuierlichen HF-Signale mittels der zweiten Gruppe von HF-Spulen empfangen. Insbesondere erfahren dabei die kontinuierlichen HF-Signale durch das Untersuchungsobjekt eine Modulation, insbesondere eine Modulation der Phase und/oder eine Modulation der Amplitude, wodurch die modulierten kontinuierlichen HF-Signale aus dem Untersuchungsobjekt generiert werden. In einem zusätzlichen Schritt wird ein Bild des Untersuchungsobjektes basierend auf den modulierten kontinuierlichen HF-Signalen bestimmt. Insbesondere werden aus den modulierten kontinuierlichen HF-Signalen Bilddaten bestimmt, auf welchem das Bild des Untersuchungsobjektes basiert. Somit werden die modulierten kontinuierlichen HF-Signale derart ausgewertet, dass ein Bild des Untersuchungsobjektes entsprechend einer EIT-Technik generiert werden kann. Dabei werden das Senden der kontinuierlichen HF-Signale und das Empfangen der modulierten kontinuierlichen HF-Signale simultan zu dem Empfangen der MR-Signale durchgeführt.

Die Amplituden der kontinuierlichen HF-Signale haben die gleiche Größenordnung wie die Amplituden der MR-Signale, welche aus dem Untersuchungsobjekt empfangen werden. Insbesondere können sie gleich groß, oder im Wesentlichen gleich groß sein wie die Amplituden der MR-Signale, so dass die Spins des Untersuchungsobjektes und die MRT-Messung nicht beeinflusst werden. Die MRT-Messung wird von zu großen Amplituden gestört durch zwei Mechanismen. Zum einen, wenn die Amplitude der kontinuierlichen HF-Signale groß genug wird um die Spins anzuregen (ca. 4-6 Größenordnungen, mV->kV), zum anderen, wenn sie groß genug ist um die MRT-Signaldetektion durch Sättigung bzw. Verlust der Signaltreue des Empfängers zu stören. Die Amplituden der MR-Signale können je nach Objekt und Messsequenz um mehrere Größenordnungen schwanken. Daher kann es vorteilhaft sein, ein "maximales detektierbares MR-Signal" zu spezifizieren und dann die Größenordnung des PT-Signals kleiner gleich diesem Signal zu wählen, z.B. gleich groß bis Faktor 100 (2 Größenordnungen, 40dB) kleiner. Zu kleine PT-Amplituden haben den Nachteil, dass das SNR der PT-Messung sinkt.

Die Amplituden können im mV-Bereich liegen, wohingegen die Amplituden des HF-Pulses im kW-Bereich liegen. Insbesondere kann durch die vergleichbare Größe der Amplituden ausgeschlossen werden, dass durch die kontinuierlichen HF-Signale die Spins des Untersuchungsobjektes beeinflusst und damit die MR-Messung gestört wird. Zusammenfassend haben die HF-Signale durch die Ströme mit geringen Amplituden, welche notwendig sind, um im signaltreuen, nicht-gesättigten Regime der MR-Empfänger zu bleiben, vernachlässigbare Auswirkungen auf die Spin-Dynamik. Dadurch kann das erfindungsgemäße Verfahren unabhängig zur MR-Messung, d.h. ohne die MR-Messung zu adaptieren, unabhängig und simultan eine EIT-Messung durchführen.

Die Hardware zum Empfangen der MR-Signale und der modulierten kontinuierlichen HF-Signale kann eine nichtlineare Charakteristik haben, die jedoch durch die Software kompensiert werden kann, wodurch in Kombination eine lineare Auswertung der empfangenen Signale ermöglicht wird. Weiter erhält das erfindungsgemäße Verfahren den angelegten Strom direkt nach dem Durchlaufen des Objektes, anstatt Änderungen im Spin-Signal zu detektieren.

Das erfindungsgemäße Verfahren benötigt keine zusätzlichen Messkreise oder Schaltungen bis auf die für die MR-Messung notwendige Hardware, sondern führt den Empfang und die Auswertung der HF-Signale zur Durchführung einer EIT rein mittels zusätzlicher Software aus. Die einzige Hardware, die benötigt wird, ist ein oder mehrere PT-Sender der MR-Anlage, welche bereits für die MR-Untersuchung vorhanden sind, d.h. kleine, preiswerte HF-Spulen, die einfach zu integrieren oder zu platzieren sind. Die PT-Sender können in einer Ausführungsform drahtlos sein. Vorteilhaft können die HF-Spulen in eine Lokalspule der MR-Anlage, daher in eine der MR-Signale empfangenden HF-Spulen eingebaut werden und somit die schon vorhandene Energieversorgung und gegebenenfalls die Signalgenerationsfähigkeit des MR nutzen. Zudem sind keine separaten Gehäuse oder Kabel für derartig ausgebildete PT-Generatorspulen notwendig.

Insbesondere kann auf diese Weise eine reine Softwarelösung auf einem MR-System mit integrierter Pilotton-Navigation bereitgestellt werden. Dadurch kann EIT auf den Markt gebracht werden, auch wenn nur vereinzelte medizinische Applikationen existieren, sodass die medizinische Versorgung von Patienten verbessert wird. Die enge Verbindung mit der MR-Bildgebung erhöht die Qualität der EIT Ergebnisse und öffnet potentiell neue Anwendungen. Insbesondere kann EIT ohne zusätzliche Patientenpräparation parallel mit der MR-Messung durchgeführt werden. Wie von der Kombination anderer Modalitäten (CT-PET, MR-PET) bekannt, erhöht sich damit die Spezifizität der Ergebnisse.

Weiter stören die kontinuierlichen HF-Signale die Spins nicht, da ihre Amplitude mit der Amplitude des MR-Signals vergleichbar groß ist und ihr schmaler Frequenzgehalt selten (oder nie) mit der lokalen Larmor-Frequenz übereinstimmt. Das erforderliche Signal-to-Noise Verhältnis (SNR) der PT-Messung wird durch eine schmalbandige Lock-in-Detektion im digitalen Bereich über einen im Vergleich zu makroskopischen physiologischen Prozessen kurzen Zeitraum sichergestellt. Das erfindungsgemäße Verfahren kann somit dazu verwendet werden, das RFIM-Verfahren auf einem Standard-MR-Scanner mit keinen oder nur minimalen Hardware-Änderungen zu implementieren.

Die EIT-basierte Gewebecharakterisierung kann für medizinische Zwecke eingesetzt werden. Durch die Verwendung von Frequenzen nahe der Larmor-Frequenz im Scanner, wie MR-RFIM, ist eine zusätzliche Nutzung möglich: EIT-Ergebnisse können dann für die patientenspezifische Modellierung von B1 verwendet werden, um MRT-Optimierungen wie SAR-Berechnungen oder PTX-Anpassungen zu unterstützen.

Dadurch wird eine eine schnellere und effizientere EIT-Bildgebung mit Hilfe einer MR-Anlage ermöglicht, welche unter Nutzung der Hardware und eines MR-Systems simultan zu einer MR-Messung durchgeführt werden kann und die MR-Messung nicht beeinflusst, sowie von der MR-Messung nicht beeinflusst wird. Das erfindungsgemäße Verfahren stellt einen geringen Zeit- und Personalaufwand für eine Untersuchung bereit, und damit eine verbesserte Patientenversorgung mit geringeren Kosten im Vergleich zu herkömmlichen EIT-Verfahren. Das erfindungsgemäße Verfahren verbessert somit die Qualität herkömmlicher EIT-Messungen, und ermöglicht damit bestehende Anwendungen zu stabilisieren und neue Anwendungen, indem qualitativ hochwertige und echtzeitfähige Modelleingaben für die Bestimmung von EIT-Bildern zur Verfügung gestellt werden.

Die Frequenz oder die Frequenzen der kontinuierlichen HF-Signale können so gewählt sein, dass die Resonanzbedingung für die Anregung von Kernspins nicht eintritt, und somit die Kernspins des Untersuchungsobjektes nicht angeregt werden, und auch die Anregung der Kernspins durch den HF-Puls nicht beeinflusst wird.

Die Frequenz, oder die Frequenzen, der kontinuierlichen HF-Signale kann außerhalb des Frequenzbereichs der MR-Signale aus dem Untersuchungsobjekt liegen, und können innerhalb eines Empfangsbereichs der zweiten Gruppe von HF-Spulen liegen. Die kontinuierlichen HF-Signale können ein monofrequentes HF-Signal umfassen, und die kontinuierlichen HF-Signale können ein HF-Signal mit konstanter Amplitude umfassen.

Durch das Senden eines monofrequenten HF-Signals, insbesondere mit konstanten Amplituden und mit Frequenzen außerhalb der Resonanzbedingung kann das Senden vorteilhaft simultan zu der MR- Messung durchgeführt werden. Weiterhin sind keine zusätzlichen Anforderungen an die Hardware eines bestehenden MR-Systems mit Pilotton-Navigation erforderlich. Die Trennung der Empfangsfrequenzen der MR-Messung und der EIT-Messung ermöglicht eine einfache Trennung der jeweiligen Daten, und eine geringe gegenseitige Beeinflussung der Messungen, daher eine verringerte Fehlerquote. Somit können die modulierten kontinuierlichen HF-Signale zum Bestimmen von EIT-Bildinformationen besonders einfach von den bestehenden Empfangsspulen des MR-Systems empfangen werden. Die mindestens eine HF Spule, welche zum Senden der HF-Signale verwendet wird, kann eine in der MR-Anlage vorhandene Pilotton-Navigator-Spule sein. Durch das Verwenden der bereits vorhandenen Pilottonspule muss keine zusätzliche Hardware in das MR-System eingebracht werden, um eine EIT-Messung durchzuführen.

Die mindestens eine HF-Spule, welche zum Senden der kontinuierlichen HF-Signale verwendet wird, kann in eine der HF-Spulen der zweiten Gruppe von HF-Spulen integriert sein, und die dort schon vorhandene Energieversorgung und Signalgenerationsfähigkeit, sowie das Gehäuse und Verkabelung nutzen. Die mindestens eine HF-Spule zum Senden der kontinuierlichen HF-Signale kann nicht in eine der HF-Spulen der ersten oder zweiten Gruppe von HF-Spulen integriert sein, und im Vergleich zu diesen HF-Spulen näher am Untersuchungsobjekt platziert sein. Die mindestens eine HF Spule kann näher als jede der HF-Spulen der ersten und zweiten Gruppe von HF Spulen am Untersuchungsobjekt platziert sein.

Dadurch, dass die mindestens eine HF Spule, welche zum Senden der kontinuierlichen HF-Signale verwendet wird, nicht in eine HF-Spule des MR-Messverfahrens integriert ist, besteht eine größere Freiheit in der Anordnung der mindestens einen HF-Spule. Insbesondere kann die Qualität der EIT-Messung verbessert werden, indem die mindestens eine HF-Spule näher am Untersuchungsobjekt und in manchen Fällen auch näher an bestimmten Regionen von Interesse des Untersuchungsobjektes, etwa bestimmten Organen einer Untersuchungsperson, angeordnet werden. Die simultane Durchführung des EIT-Messverfahrens neben dem MR-Messverfahren wird dadurch weiter vereinfacht und die Qualität der EIT-Untersuchung erhöht.

Das Bestimmen eines Bildes des Untersuchungsobjektes kann ein Bestimmen einer Phase der modulierten kontinuierlichen HF-Signale umfassen. Dabei kann eine absolute Phase relativ zu den gesendeten kontinuierlichen HF-Signalen bestimmt werden. Weiter kann eine relative zeitliche Phasenänderung der modulierten kontinuierlichen HF-Signale erfasst werden. Die Erfassung der Phase der modulierten kontinuierlichen HF Signale ermöglicht ein verbessertes EIT-Verfahren, wobei keine zusätzliche Hardware in die MR-Anlage eingebracht werden muss. Insbesondere müssen keine zusätzlichen Messschaltkreise, welche beispielsweise zur Messung und Überwachung einer Impedanz der HF-Spulen der ersten oder der zweiten Gruppe von HF-Spulen ausgebildet sind, an der bestehenden MR-Anlage zur Durchführung der EIT-Messung bereitgestellt werden.

Das Bestimmen eines Bildes des Untersuchungsobjektes kann unter Verwenden der empfangenen MR-Signale erfolgen, insbesondere kann dadurch das zusätzliche Verwenden von Informationen aus der MR-Messung eine vereinfachte und qualitativ höherwertige Bestimmung des EIT-Bildes bereitgestellt werden.

Das Verfahren kann weiter ein Bestimmen eines Frequenzbereiches der empfangenen MR-Signale aus dem Untersuchungsobjekt, und ein Senden der kontinuierlichen HF-Signale außerhalb des bestimmten Frequenzbereiches der MR-Signale umfassen. Durch das Bestimmen des Frequenzbereichs der empfangenen MR Signale können die kontinuierlichen HF Signale mit Frequenzen gesendet werden, welche nicht in den MR-Signalen enthalten sind. Insbesondere können die kontinuierlichen Signale etwa ein MHz neben dem Bereich der MR-Signale liegen. Dadurch ist eine Trennung der Datensignale der beiden bildgebenden Verfahren möglich, wodurch das EIT-Verfahren schneller und fehlerfreier simultan zu der MR-Messung durchgeführt werden kann.

Die kontinuierlichen HF-Signale können mittels mehrerer HF-Spulen gesendet werden, das heißt die mindestens eine HF Spule, welche zum Senden der kontinuierlichen HF Signale verwendet wird, kann mehrere HF-Spulen umfassen.

Dabei können die mehreren HF-Spulen gleichzeitig verschiedene kontinuierliche HF-Signale aussenden, welche sich in einer oder mehreren Sendeeigenschafen unterscheiden. Die Sendeeigenschaften sind ausgewählt aus der Gruppe umfassend der Frequenz, ihrer relativen Phasen, der Amplituden, der elektromagnetischen Polarisationsrichtung oder der Signalmodulation.

Die mehreren HF-Spulen können sequenziell HF-Signale aussenden. Die Sendeeigenschaften können sequentiell variieren.

Auch eine Kombination von sequenziell sendenden HF Spulen und sich in ihrer Frequenz, ihrer elektromagnetischen Polarisationsrichtung oder ihrer so Signalmodulation unterscheidenden kontinuierliche HF-Signale kann erfindungsgemäß bereitgestellt werden. Durch mehrere kontinuierliche HF-Signale sendende HF-Spulen wird die Informationsdichte des EIT-Verfahrens erhöht, es werden daher genauere und mehr Informationen gesammelt, welche zur Bestimmung des EIT-Bildes verwendet werden können. Dadurch wird eine schnellere und genauere Abbildung des Untersuchungsobjektes durch das EIT-Verfahren ermöglicht.

Mindestens eine der mehreren HF-Spulen kann während der EIT-Messsequenz im Raum relativ zu dem Untersuchungsobjekt verschoben werden. Das Verschieben ermöglicht ein Messen von zusätzlichen Informationen, und erhöht die Qualität der EIT-Bildgebung zusätzlich.

Das Untersuchungsobjekt kann eine dynamische Änderung aufweisen, wobei bei dem Bestimmen des Bildes des Untersuchungsobjektes nur die Variationen der modulierten kontinuierlichen HF Signale basierend auf der dynamischen Änderung berücksichtigt werden. Die Datenmenge der zu verarbeitenden EIT-Messdaten kann somit reduziert werden, wobei lediglich die wesentlichen Informationen bezüglich der interessierenden dynamischen Änderung für die EIT-Bildgebung berücksichtigt werden. Dies führt zu einem schnelleren Messergebnis und ermöglicht eine schnellere Auswertung und Bilderzeugung in dem EIT-Messverfahren.

Die modulierten kontinuierlichen HF-Signale können mittels HF-Spulen aus der zweiten Gruppe von HF-Spulen empfangen werden. Diese Spulen werden neben der neben dem Empfang von EIT-Signalen auch zum Empfang von MR-Signalen verwendet, insbesondere werden die genannten Signale parallel oder simultan zueinander aufgenommen. Die EIT-Signale, d.h. die kontinuierlichen modulierten HF-Signale werden infolge zur Bestimmung eines Bildes des Untersuchungsobjektes ausgewertet.

Gemäß einem weiteren Aspekt der Erfindung wird eine MR-Anlage zum Durchführen einer Elektrische-Impedanz-Tomographie (EIT) an einem Untersuchungsobjekt bereitgestellt. Die MR-Anlagen umfasst eine Vielzahl von HF Spulen, eine MR-Steuereinheit und eine Speichereinheit. Dabei ist die MR Anlage ist derart ausgebildet, dass sie bei der Ausführung der Steuerinformationen in der MR-Steuereinheit die folgenden Schritte ausführt.

In einem ersten Schritt wird ein HF-Puls mittels einer ersten Gruppe von HF-Spulen zum Anregen von Spins in dem Untersuchungsobjekt gesendet. In einem weiteren Schritt werden MR-Signale aus dem Untersuchungsobjekt basierend auf dem HF-Puls mittels einer zweiten Gruppe von HF-Spulen empfangen. In einem zusätzlichen Schritt werden kontinuierliche HF-Signale mittels mindestens einer HF-Spule gesendet, welche Amplituden in der Größenordnung der Amplituden der MR-Signale aufweisen. In einem weiteren Schritt werden modulierte kontinuierliche HF-Signale aus dem Untersuchungsobjekt in Antwort auf die kontinuierlichen HF-Signale mittels der zweiten Gruppe von HF-Spulen empfangen. Insbesondere erfahren dabei die kontinuierlichen HF-Signale durch das Untersuchungsobjekt eine Modulation, insbesondere eine Modulation der Phase und/oder eine Modulation der Amplitude oder der Frequenz, wodurch die modulierten kontinuierlichen HF-Signale aus dem Untersuchungsobjekt generiert werden. In einem zusätzlichen Schritt wird ein Bild des Untersuchungsobjektes basierend auf den modulierten kontinuierlichen HF-Signalen bestimmt. Insbesondere werden aus den modulierten kontinuierlichen HF-Signalen Bilddaten bestimmt, auf welchem das Bild des Untersuchungsobjektes basiert. Somit werden die modulierten kontinuierlichen HF-Signale derart ausgewertet, dass ein Bild des Untersuchungsobjektes entsprechend einer EIT-Technik generiert werden kann. Dabei werden das Senden der kontinuierlichen HF-Signale und das Empfangen der modulierten kontinuierlichen HF-Signale simultan zu dem Empfangen von MR-Signalen durchgeführt.

Die MR-Anlage kann ausgebildet sein, bei der Ausführung der Steuerinformationen in der MR-Steuereinheit das Verfahren entsprechend der weiteren oben beschriebenen Merkmale auszuführen.

Für eine derartige MR-Anlage zum Durchführen einer Elektrische-Impedanz-Tomographie (EIT) können technische Effekte erzielt werden, die vergleichbar sind mit den technischen Effekten, die für das Verfahren gemäß dem ersten Aspekt obenstehend beschrieben wurden.

Gemäß einem weiteren Aspekt der Erfindung wird ein Computerprogrammprodukt bereitgestellt, welches ein Programm umfasst, das direkt in einen Speicher einer MR-Steuereinheit einer MR-Anlage ladbar ist, mit Programm-Mitteln, um die Schritte des Verfahrens entsprechend den unter dem ersten Aspekt der Erfindung beschriebenen Merkmale auszuführen, wenn das Programm in der MR-Steuereinheit der MR-Anlage ausgeführt wird.

Gemäß einem weiteren Aspekt der Erfindung wird ein elektronisch lesbarer Datenträger mit darauf gespeicherten elektronisch lesbaren Steuerinformationen bereitgestellt, welche derart ausgestaltet sind, dass sie bei Verwendung des Datenträgers in einer MR-Steuereinheit einer MR-Anlage das Verfahren entsprechend den unter dem ersten Aspekt der Erfindung beschriebenen Merkmalen durchführen.

Die oben dargelegten Merkmale und Merkmale, die nachfolgend beschrieben werden, können nicht nur in den entsprechenden explizit dargelegten Kombinationen verwendet werden, sondern auch in weiteren Kombinationen oder isoliert voneinander, ohne den Schutzumfang der vorliegenden Erfindung zu verlassen.

### Kurze Beschreibung der Zeichnungen

Die Erfindung wird nachfolgend unter Bezugnahme auf die beiliegenden Zeichnungen näher erläutert.
Figur 1 zeigt schematisch eine MR-Anlage, mit der ein Verfahren zum Durchführen einer Elektrische-Impedanz-Tomographie (EIT) erfindungsgemäß durchgeführt werden kann.
Figur 2 zeigt ein Flussdiagramm mit Schritten zur Durchführung eines Verfahrens zum Durchführen einer Elektrische-Impedanz-Tomographie (EIT) gemäß einem Ausführungsbeispiel der Erfindung.

### Detaillierte Beschreibung

Die vorliegende Erfindung betrifft ein Verfahren zum Durchführen einer Elektrische-Impedanz-Tomographie (EIT) mit Hilfe einer MR-Anlage.

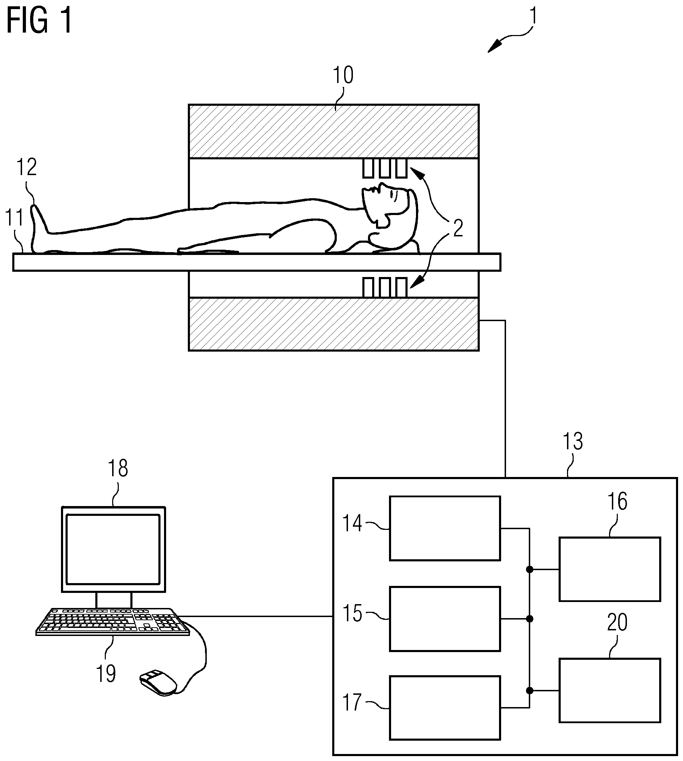

Figur 1 zeigt schematisch eine MR-Anlage, mit der solch ein Verfahren zum Durchführen einer Elektrische-Impedanz-Tomographie (EIT) erfindungsgemäß durchgeführt werden kann.

Eine Untersuchungsperson 12, oder allgemeiner ein Untersuchungsobjekt, ist in den Tunnel der Anlage gefahren. Die Magnetresonanzanlage weist einen Magneten 10 zur Erzeugung eines Grundfeldes B0 auf, wobei die auf einer Liege 11 angeordnete Untersuchungsperson 12 in das Zentrum des Magneten gefahren wird, um dort ortscodierte Magnetresonanzsignale aufzunehmen. Durch Einstrahlen von Hochfrequenzpulsfolgen und Schalten von Magnetfeldgradienten kann die durch das Grundfeld B0 erzeugte Magnetisierung durch Auslenkung der Kernspins aus der Gleichgewichtslage gestört werden, und bei der Rückkehr der Kernspins in die Gleichgewichtslage in Empfangsspulen induzierte Ströme können in Magnetresonanz- (MR)-Signale umgewandelt werden. Die allgemeine Funktionsweise zur Erstellung von MR-Bildern und die Detektion der Magnetresonanzsignale sind dem Fachmann bekannt, sodass auf eine detaillierte Erläuterung hiervon verzichtet wird. Im Sinne der vorliegenden Erfindung bedeutet die Ausdrücke Spule, HF-Spule, MR Sende- oder Empfangsspule typischerweise nicht gewickelte Leiter, sondern bei MR-Anlagen einfache Leiterschleifen.
Die Magnetresonanzanlage 1 weist eine Vielzahl von HF-Spulen auf, insbesondere umfassen die HF Spulen eine erste Gruppe von HF-Spulen, welche dazu ausgebildet sind, mindestens einen HF-Puls zum Anregen von Kernspins in dem Untersuchungsobjekt auszusenden, und eine zweite Gruppe von HF-Spulen, welche ausgebildet sind, MR-Signale aus dem Untersuchungsobjekt zu empfangen. Weiter umfasst die MR-Anlage mindestens eine HF-Spule, welche ausgebildet ist, einen Pilot-Tone (PT)-Navigator Signal auszusenden. Das PT-Verfahren, sowie die zugehörigen PT-Generatoren bzw. PT-Signalsender sind in den in der Einleitung der Beschreibung genannten Druckschriften näher beschrieben, auf welche hiermit verwiesen wird.

Die Magnetresonanzanlage 1 weist weiterhin eine MR-Steuereinheit 13 auf, die zur Steuerung des MR-Geräts verwendet wird. Die zentrale MR-Steuereinheit 13, welche derart ausgebildet ist, dass sie das unten beschriebene Verfahren zum Durchführen einer Elektrische-Impedanz-Tomographie (EIT) durchführen kann, weist eine Gradientensteuerung 14 zur Steuerung und Schaltung der Magnetfeldgradienten auf, und eine HF-Steuerung 15 zur Steuerung und Einstrahlung der HF-Pulse zur Auslenkung der Kernspins aus der Gleichgewichtlage und zum Senden der kontinuierlichen HF-Signale. In einer Speichereinheit 16 können beispielsweise die für die Aufnahme der MR-Bilder und der EIT-Bilder notwendigen Bildgebungssequenzen abgespeichert werden, sowie alle Programme, die zum Betrieb der MR-Anlage sowie zum Durchführen einer Elektrische-Impedanz-Tomographie entsprechend dem erfindungsgemäßen Verfahren notwendig sind. Eine Aufnahmeeinheit 17 steuert die Bildaufnahme und steuert damit in Abhängigkeit von den gewählten Bildgebungssequenzen die Abfolge der Magnetfeldgradienten und HF-Pulse, die Empfangsintervalle von MR-Signalen, und das Senden und Empfangen der kontinuierlichen HF-Signale. Somit steuert die Aufnahmeeinheit 17 auch die Gradientensteuerung 14 und die HF-Steuerung 15. Somit steuert die Aufnahmeeinheit 17 simultan zum dem MR-Verfahren auch das EIT-Verfahren, und daher auch die HF-Spulen, welche zum Senden von kontinuierlichen HF-Signalen verwendet werden, sowie die HF-Spulen, welche zum Empfangen der modulierten kontinuierlichen HF-Signale verwendet werden.

In einer Recheneinheit 20 können MR-Bilder und EIT-Bilder berechnet werden, die auf eine Anzeige 18 angezeigt werden können, wobei eine Bedienperson über eine Eingabeeinheit 19 die MR-Anlage 1 bedienen kann. Die Speichereinheit 16 kann Bildgebungssequenzen und Programmmodule aufweisen, die bei Ausführung in der Recheneinheit 20 von einem der gezeigten Module, das erfindungsgemäße Verfahren für eine Elektrische-Impedanz-Tomographie durchführen, wie nachfolgend im Detail erläutert wird. Insbesondere speichert die Speichereinheit 16 dazu von der MR-Steuereinheit 13 ausführbare Steuerinformationen. Insbesondere ist die Aufnahmeeinheit 17 derart ausgebildet, dass sie das nachfolgend beschriebene Verfahren zum Durchführen einer Elektrische-Impedanz-Tomographie (EIT) durchführen kann.

Vorhandenen Hardware- und Software-Features der MR-Anlage 1 werden ausgenutzt, um EIT/RFIM mit dem MRT-Scanner durchzuführen, wie folgend beschrieben wird.

Erfindungsgemäß ist die MR-Anlage 1 der Figur 1 derart ausgebildet, dass sie bei der Ausführung der Steuerinformationen in der MR-Steuereinheit 13 ein Magnetfeld an eine Untersuchungsperson 12 anlegt. Weiter werden Kernspins in der Untersuchungsperson durch einen HF-Puls anregt, während das Magnetfeld angelegt ist. Weiterhin werden MR-Signale aus der Untersuchungsperson gemessen. Simultan dazu werden kontinuierliche HF-Signale mittels mindestens einer HF-Spule in den Tunnel der MR-Anlage 1 gesendet, welche Amplituden in der Größenordnung der Amplituden der empfangenen MR-Signale aufweisen.

Dabei orientiert sich das EIT-Verfahren an dem zuvor beschriebenen Pilot-Tone (PT)-Navigations-Framework. Aus der Magisterarbeit "Informationsgehalt eines neuartigen MR-Navigators in Bezug auf physiologische Aktivitäten" von Lea Schröder, Friedrich-Alexander-Universität Erlangen-Nürnberg, 2015, ist bekannt, dass der PT-Navigator empfindlich auf mehrdimensionale Atmungsbewegungen, Herzkontraktionen und Extremitätenbewegungen reagiert. Weiterhin ist daraus bekannt, dass das Bewegungsvolumen, das zur Modulation des PT-Signals beiträgt, in etwa durch die Entfernung zwischen Sender und Empfänger begrenzt ist.

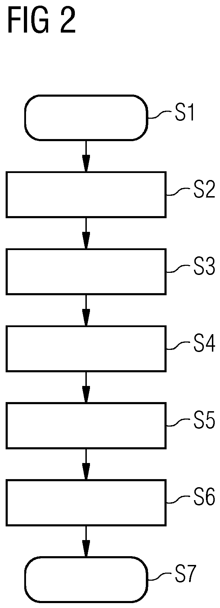

Figur 2 zeigt ein Flussdiagramm mit Schritten zur Durchführung eines Verfahrens zum Durchführen einer Elektrische-Impedanz-Tomographie (EIT) gemäß einem Ausführungsbeispiel der Erfindung.

Das Verfahren beginnt in Schritt S1. In Schritt S2 wird ein HF-Puls mittels einer ersten Gruppe von HF-Spulen 2 zum Anregen von Spins in dem Untersuchungsobjekt 12 gesendet. In Schritt S3 werden MR-Signale aus dem Untersuchungsobjekt 12 basierend auf dem HF-Puls mittels einer zweiten Gruppe von HF-Spulen 2 empfangen. Dabei liefert die MR-Bildgebung exakte Modellgrenzen: Körpergeometrie, Gewebeverteilung (z.B. Weichteil-/Hohlraum-/Fettverteilung, Fett-/Wassergehalt pro Voxel, Organseparation aus Segmentierung und/oder Benutzereingaben, Faserorientierung aus DTI), dynamische Parameter (z.B. Herzkontraktion, Atemdeformation).

In Schritt S4 werden kontinuierliche HF-Signale, welche Amplituden in der Größenordnung der Amplituden der MR-Signale aufweisen, mittels mindestens einer HF-Spule 2 gesendet. Die Frequenz oder die Frequenzen der kontinuierlichen HF-Signale sind so gewählt, dass die Resonanzbedingung für die Anregung von Kernspins in der Untersuchungsobjekts 12 nicht eintritt, und somit die Kernspins des Untersuchungsobjektes 12 nicht angeregt werden, und auch die Anregung der Kernspins durch den HF-Puls nicht beeinflusst wird. Die Amplituden sind dabei so klein gewählt, dass sie im Bereich der MR-Signale liegen, und somit verhindern, dass die MR-Bildgebung durch die kontinuierlichen HF-Signale beeinflusst wird. Dabei werden die Ströme nicht über einen galvanischen Kontakt, sondern über einen Luftspalt eines Pilot-Tone-Senders als monofrequentes Signal mit konstanter Amplitude in den Körper bzw. das Untersuchungsobjekt 12 eingekoppelt.
Die HF-Signale haben durch die Ströme mit geringen Amplituden, welche notwendig sind, um im signaltreuen, nicht-gesättigten Regime der MR-Empfänger zu bleiben, und ihren schmalen Frequenzgehalt, welcher die Resonanzbedingung vermeidet, vernachlässigbare Auswirkungen auf die Spin-Dynamik. Dadurch kann das erfindungsgemäße EIT-Verfahren simultan, d.h. unabhängig zur MR-Messung, ohne die MR-Messung zu adaptieren, durchgeführt werden.

In Schritt S5 werden modulierte kontinuierliche HF-Signale aus dem Untersuchungsobjekt 12 in Antwort auf die kontinuierlichen HF-Signale mittels der zweiten Gruppe von HF-Spulen 2 empfangen. Insbesondere erfahren dabei die kontinuierlichen HF-Signale durch das Untersuchungsobjekt 12 eine Modulation, insbesondere eine Modulation der Phase und/oder eine Modulation der Amplitude, wodurch die modulierten kontinuierlichen HF-Signale durch Interaktion mit dem Untersuchungsobjekt 12 generiert werden. Dabei werden Ströme über einen Luftspalt mit den MR-Lokal-Empfangsspulen empfangen und phasenkohärent in den MR-Empfängern detektiert.

Die Bandbreite der MR-Signale zur Zeit der Anregung entspricht ungefähr der Bandbreite des Anregungspulses. Jedoch werden die HF-Signale nur gemessen, wenn die MR-Anlage 1 empfangsbereit ist, d.h. zwischen den Anregungspulsen, insbesondere in den Zeiträumen, in welchen die MR-Signale aus dem Untersuchungsobjekt 12 empfangen werden. Im Empfangszeitraum wird die Bandbreite der MR-Signale durch angelegten B0-Kodiergradienten bestimmt. Sie kann grösser oder kleiner als die Bandbreite des Anregungspulses sein. Typischer Werte für die Bandbreite des Anregungspulses sind 1 kHz für den MR-Anregungspuls, und 250 kHz für das MR-Signal.
Für die MR-Messung gibt es eine zeitlich getrennte Abfolge von Anregung mit HF-Puls und Empfang von MR-Signalen. Für die kontinuierlichen HF-Signale ist das nicht der Fall. Hierbei wird zu einem Zeitpunkt das kontinuierliche HF-Signal detektiert, das zu diesem Zeitpunkt anliegt. Da das MR-System nur zwischen den Anregungspulsen empfangsbereit ist, kann das Senden der kontinuierlichen HF-Signale durchgehend während des gesamten MR-Messvorgangs, d.h. während des Senden des HF-Pulses und des Empfanges der MR-Signale erfolgen, und es ist anderseits auch möglich die Erzeugung der kontinuierlichen HF-Signale auf diese Zeiträume, wenn das MR-System empfangsbereit ist, zu beschränken.

Insbesondere werden das Senden der kontinuierlichen HF-Signale in Schritt S4 und das Empfangen der modulierten kontinuierlichen HF-Signale in Schritt S5 simultan zu dem Empfangen von MR-Signalen in Schritt S3 durchgeführt. Dabei kann die MR-Bildgebung auch in Echtzeit während der EIT-Messung Informationen über sich ändernde Randbedingungen liefern.

Dadurch wird das MR- und das EIT-Messverfahren simultan und unabhängig voneinander durchgeführt und die Auswertung der MR- und der EIT-Messdaten Daten können zeitgleich in der MR-Anlage erfolgen. Dadurch können die Messdaten bzw. die ausgewerteten Daten beider Messverfahren im jeweiligen anderen Verfahren verwendet werden, um die Genauigkeit des Bildgebenden Verfahrens zu erhöhen.

In Schritt S6 wird ein Bild des Untersuchungsobjektes 12 basierend auf den modulierten kontinuierlichen HF-Signalen bestimmt. Insbesondere werden aus den modulierten kontinuierlichen HF-Signalen Bilddaten bestimmt, auf welchen das Bild des Untersuchungsobjektes basiert. Somit werden die modulierten kontinuierlichen HF-Signale derart ausgewertet, dass ein Bild des Untersuchungsobjektes entsprechend einer EIT-Technik aus den Bilddaten der kontinuierlichen HF-Signale generiert wird. Dabei erfolgt die Modellinversion mit Hilfe der MR-Bildrekonstruktionshardware und kann in die MR-Bildrekonstruktionssoftware integriert werden. Insbesondere können die in Schritt S3 bestimmten Modellgrenzen verwendet werden, um das EIT-Verfahren genauer und schneller durchzuführen. Das Verfahren endet in Schritt S7.

Aus den oben beschriebenen Ausführungsbeispielen können die folgenden Schlüsse gezogen werden:
In einem Ausführungsbeispiel können in einem zusätzlichen Schritt des Verfahrens EIT-Bilder mit der Standard-MR-Systemsoftware dargestellt werden.

In einem Ausführungsbeispiel können nicht in die MR-Spulen integrierte Pilot-Ton-Sender an für die EIT eines bestimmten Organs optimalen Positionen platziert werden.

In einem Ausführungsbeispiel können mehrere Pilot-Ton-Sender an unterschiedlichen Positionen platziert werden um die Information in der EIT-Messung erhöhen und die Modellinversion zu stabilisieren. Die Signalbeiträge der einzelnen Sender können sich in ihrer Frequenz und elektromagnetischen Polarisationsrichtung und evtl. Signalmodulation unterscheiden und in den empfangenen Daten mithilfe dieser Eigenschaften separiert werden.

In einem Ausführungsbeispiel mit mehreren parallel betriebenen Sendern kann eine Reihe von Sendern mit gleicher Frequenz sequentiell aktiviert werden oder ein Sender im Raum verschoben werden um die Datenmenge zu erhöhen.

In einem Ausführungsbeispiel ist das Untersuchungsobjekt 12 nicht statisch, sondern hat eine bekannte Dynamik die seine elektrischen Eigenschaften moduliert. Somit kann das Inversionsproblem vereinfacht werden indem man nur die Variation der Messung mit der Dynamik betrachtet und statische Anteile des Modells wegfallen. Beispielsweise kann die EIT-Messung über den Herzschlag zeitaufgelöst vorgenommen werden und nur die Differenz der Signale zwischen Systole und Diastole ausgewertet werden unter der Annahme, dass die Dynamik auf das Herz beschränkt ist, bzw. unter Benutzung zeitlich aufgelöster MR-Informationen. Ähnliches gilt für die Atmungsbewegung oder den pulsatilen Blutfluss im Gewebe.

In einem Ausführungsbeispiel kann EIT-basierte Gewebecharakterisierung für medizinische Zwecke eingesetzt werden. Durch die Verwendung von Frequenzen nahe der Larmor-Frequenz im Scanner, wie MR-RFIM, ist eine zusätzliche Nutzung möglich: EIT-Ergebnisse können dann für die patientenspezifische Modellierung von B1 verwendet werden, um MRT-Optimierungen wie SAR-Berechnungen oder PTX-Anpassungen zu unterstützen.

Zusammenfassend wird ein Verfahren zum Durchführen einer Elektrische-Impedanz-Tomographie (EIT) mittels einer MR-Anlage bereitgestellt, wobei simultan zu dem Empfang der MR-Signale kontinuierliche HF-Signale für eine EIT mittels mindestens einer HF-Spule gesendet werden, und durch das Untersuchungsobjekt modulierte kontinuierliche HF-Signale durch die MR-Spulen empfangen werden. Ein Bild des Untersuchungsobjektes wird basierend auf den modulierten kontinuierlichen HF-Signalen mittels einer EIT-Technik bestimmt. In einem Ausführungsbeispiel wird durch das Vermeiden der Resonanzfrequenzen und das Verwenden eines monofrequenten kontinuierlichen HF-Signals mit kleiner, konstanter Amplitude eine Wechselwirkung des EIT-Verfahrens mit dem MR-Verfahren ausgeschlossen.

Durch das erfindungsgemäße Verfahren kann auf einem MR-System mit integrierter Pilotton-Navigation eine verbesserte EIT-Bildgebung simultan zu der MR-Bildgebung bereitgestellt werden. Die enge Verbindung mit der MR-Bildgebung erhöht die Qualität der EIT-Ergebnisse, insbesondere kann EIT dadurch ohne zusätzliche Patientenpräparation parallel mit der MR-Messung durchgeführt werden. Somit wird eine eine genauere und kostengünstigere Patientenversorgung im Vergleich zu herkömmlichen EIT-Untersuchungsverfahren bereitstellt.

## Patentansprüche

1. Verfahren zum Durchführen einer Elektrische-Impedanz-Tomographie (EIT) an einem Untersuchungsobjekt (12) mit Hilfe einer MR-Anlage (1) umfassend eine Vielzahl von HF-Spulen (2), mit den folgenden Schritten:
- Senden eines HF-Pulses mittels einer ersten Gruppe von HF-Spulen (2) zum Anregen von Spins in dem Untersuchungsobjekt (12);
- Empfangen von MR-Signalen aus dem Untersuchungsobjekt (12) basierend auf dem HF-Puls mittels einer zweiten Gruppe von HF-Spulen (2);
- Senden von kontinuierlichen HF-Signalen, welche Amplituden in der Größenordnung der Amplituden der MR-Signale aufweisen, mittels mindestens einer HF-Spule (2);
- Empfangen von modulierten kontinuierlichen HF-Signalen aus dem Untersuchungsobjekt (12) in Antwort auf die kontinuierlichen HF-Signale mittels der zweiten Gruppe von HF-Spulen (2); und
- Bestimmen eines Bildes des Untersuchungsobjektes (12) basierend auf den modulierten kontinuierlichen HF-Signalen;
wobei das Senden der kontinuierlichen HF-Signale und das Empfangen der modulierten kontinuierlichen HF-Signale simultan zu dem Empfangen der MR-Signale durchgeführt werden.

2. Verfahren nach Anspruch 1, wobei die Frequenz oder die Frequenzen der kontinuierlichen HF-Signale so gewählt sind, dass die Resonanzbedingung für die Anregung von Kernspins in dem Untersuchungsobjekt (12) nicht eintritt.

3. Verfahren nach einem der vorhergehenden Ansprüche, wobei die Frequenz oder die Frequenzen der kontinuierlichen HF-Signale außerhalb des Frequenzbereichs der MR-Signale aus dem Untersuchungsobjekt (12) und innerhalb eines Empfangsbereichs der zweiten Gruppe von HF-Spulen (2) liegt bzw. liegen.

4. Verfahren nach einem der vorhergehenden Ansprüche, wobei die kontinuierlichen HF-Signale ein monofrequentes HF-Signal mit konstanter Amplitude umfassen.

5. Verfahren nach einem der vorhergehenden Ansprüche, wobei die mindestens eine HF-Spule (2), welche zum Senden der HF-Signale verwendet wird, eine HF-Spule (2) zum Senden eines Pilot-Tone-Navigatorsignals ist.

6. Verfahren nach einem der vorhergehenden Ansprüche, wobei die mindestens eine HF-Spule, welche zum Senden der kontinuierlichen HF-Signale verwendet wird, in eine der HF-Spulen (2) der zweiten Gruppe von HF-Spulen (2) integriert ist.

7. Verfahren nach einem der Ansprüche 1 bis 5, wobei die mindestens eine HF-Spule (2), welche zum Senden der kontinuierlichen HF-Signale verwendet wird, nicht in eine der HF-Spulen (2) der zweiten Gruppe von HF-Spulen (2) integriert ist und im Vergleich zu einer HF-Spule (2) der zweiten Gruppe von HF-Spulen (2) näher am Untersuchungsobjekt (12) platziert ist.

8. Verfahren nach einem der vorhergehenden Ansprüche, wobei das Bestimmen eines Bildes des Untersuchungsobjektes (12) ein Bestimmen einer Phase der modulierten kontinuierlichen HF-Signale umfasst, wobei eine absolute Phase relativ zu den gesendeten HF-Signalen bestimmt wird, oder eine relative zeitliche Phasenänderung der modulierten kontinuierlichen HF-Signale bestimmt wird.

9. Verfahren nach einem der vorhergehenden Ansprüche, wobei das Bestimmen eines Bildes des Untersuchungsobjektes (12) zusätzlich unter Verwenden der empfangenen MR-Signale erfolgt.

10. Verfahren nach einem der vorhergehenden Ansprüche, weiter umfassend:
- Bestimmen eines Frequenzbereiches der empfangenen MR-Signale aus dem Untersuchungsobjekt (12); und
- Senden der kontinuierlichen HF-Signale außerhalb des bestimmten Frequenzbereiches der MR-Signale.

11. Verfahren nach einem der vorhergehenden Ansprüche, wobei die kontinuierlichen HF-Signale mittels mehrerer HF-Spulen (2) gesendet werden.

12. Verfahren nach Anspruch 11, wobei die mehreren HF-Spulen (2) gleichzeitig senden und die kontinuierlichen HF-Signale der einzelnen HF-Spulen (2) sich in einer oder mehreren Sendeeigenschaften unterscheiden, umfassend ihre Frequenz, ihrer relative Phase, ihre Amplitude, ihre elektromagnetischen Polarisationsrichtung, und ihre Signalmodulation.

13. Verfahren nach Anspruch 11 oder 12, wobei die mehreren HF-Spulen (2) sequentiell senden.

14. Verfahren nach Anspruch 12 oder 13, wobei die eine oder mehrere Sendeeigenschaften sequentiell variieren.

15. Verfahren nach einem der Ansprüche 11 bis 14, wobei mindestens eine der HF-Spulen (2) im Raum verschoben wird.

16. Verfahren nach einem der vorhergehenden Ansprüche, wobei das Untersuchungsobjekt (12) eine dynamische Änderung aufweist, und wobei bei dem Bestimmen eines Bildes des Untersuchungsobjektes (12) nur die Variationen der kontinuierlichen modulierten HF-Signale basierend auf der dynamischen Änderung berücksichtigt werden.

17. Verfahren nach einem der vorhergehenden Ansprüche, wobei die Amplituden der kontinuierlichen HF-Signale im Wesentlichen gleich groß sind, wie die Amplituden der MR-Signale.

18. MR-Anlage zum Durchführen einer Elektrische-Impedanz-Tomographie (EIT) an einem Untersuchungsobjekt (12), wobei die MR-Anlage (1) eine Vielzahl von HF-Spulen (2), eine MR-Steuereinheit (13) und eine Speichereinheit (16) aufweist, wobei die Speichereinheit (16) von der MR-Steuereinheit (13) ausführbare Steuerinformationen speichert, und wobei die MR-Anlage ausgebildet ist, bei der Ausführung der Steuerinformationen in der MR-Steuereinheit (13) folgende Schritte auszuführen:
- Senden eines HF-Pulses mittels einer ersten Gruppe von HF-Spulen (2) zum Anregen von Spins in dem Untersuchungsobjekt (12);
- Empfangen von MR-Signalen aus dem Untersuchungsobjekt (12) basierend auf dem HF-Puls mittels einer zweiten Gruppe von HF-Spulen;
- Senden von kontinuierlichen HF-Signalen, welche Amplituden in der Größenordnung der Amplituden der MR-Signale aufweisen, mittels mindestens einer der HF-Spulen (2);
- Empfangen von modulierten kontinuierlichen HF-Signalen aus dem Untersuchungsobjekt (12) in Antwort auf die kontinuierlichen HF-Signale mittels der zweiten Gruppe von HF-Spulen (2); und
- Bestimmen eines Bildes des Untersuchungsobjektes (12) basierend auf den modulierten kontinuierlichen HF-Signalen;
wobei das Senden der kontinuierlichen HF-Signale und das Empfangen der modulierten kontinuierlichen HF-Signale simultan zu dem Empfangen von MR-Signalen durchgeführt werden.

19. MR-Anlage zum Durchführen einer Elektrische-Impedanz-Tomographie (EIT) an einem Untersuchungsobjekt (12), wobei die MR-Anlage eine Vielzahl von HF-Spulen (2), eine MR-Steuereinheit (13) und eine Speichereinheit (16) aufweist, wobei die Speichereinheit (16) von der MR-Steuereinheit (13) ausführbare Steuerinformationen speichert, und wobei die MR-Anlage ausgebildet ist, bei der Ausführung der Steuerinformationen in der MR-Steuereinheit (13) das Verfahren eines der Ansprüche 2 bis 17 auszuführen.

20. Computerprogrammprodukt, welches ein Programm umfasst, das direkt in einen Speicher einer MR-Steuereinheit (13) einer MR-Anlage (1) ladbar ist, mit Programm-Mitteln, um die Schritte des Verfahrens nach einem der Ansprüche 1 bis 17 auszuführen, wenn das Programm in der MR-Steuereinheit (13) der MR-Anlage (1) ausgeführt wird.

21. Elektronisch lesbarer Datenträger mit darauf gespeicherten elektronisch lesbaren Steuerinformationen, welche derart ausgestaltet sind, dass sie bei Verwendung des Datenträgers in einer MR-Steuereinheit (13) einer MR-Anlage (1) das Verfahren nach einem der Ansprüche 1 bis 17 durchführen.

## Claims

1. Method for performing electrical impedance tomography (EIT) on an object undergoing examination (12) with the aid of an MR system (1) comprising a plurality of RF coils, having the following steps:
- emitting an RF pulse by means of a first group of RF coils (2), for the purpose of exciting spin in the object undergoing examination (12);
- receiving MR signals from the object undergoing examination (12), based on the RF pulse, by means of a second group of RF coils (2);
- emitting continuous RF signals having amplitudes in the same order of magnitude as the amplitudes of the MR signals, by means of at least one RF coil (2);
- receiving modulated continuous RF signals from the object undergoing examination (12), in response to the continuous RF signals, by means of the second group of RF coils (2); and
- determining an image of the object undergoing examination (12) on the basis of the modulated continuous RF signals;
wherein emission of the continuous RF signals and receiving of the modulated continuous RF signals are performed simultaneously with receiving of the MR signals.

2. Method according to claim 1, wherein the frequency or frequencies of the continuous RF signals are selected such that the resonance condition for exciting nuclear spin in the object undergoing examination (12) does not occur.

3. Method according to one of the preceding claims, wherein the frequency or frequencies of the continuous RF signals lie(s) outside the frequency range of the MR signals from the object undergoing examination (12) and within a receiving range of the second group of RF coils (2).

4. Method according to one of the preceding claims, wherein the continuous RF signals comprise a single-frequency RF signal of constant amplitude.

5. Method according to one of the preceding claims, wherein the at least one RF coil (2) that is used to emit the RF signals is an RF coil (2) for emitting a pilot tone navigator signal.

6. Method according to one of the preceding claims, wherein the at least one RF coil that is used to emit the continuous RF signals is integrated into one of the RF coils (2) of the second group of RF coils (2).

7. Method according to one of claims 1 to 5, wherein the at least one RF coil (2) that is used to emit the continuous RF signals is not integrated into one of the RF coils (2) of the second group of RF coils (2) and is placed closer to the object undergoing examination (12) than an RF coil (2) of the second group of RF coils (2).

8. Method according to one of the preceding claims, wherein determining an image of the object undergoing examination (12) comprises determining a phase of the modulated continuous RF signals, wherein an absolute phase is determined in relation to the emitted RF signals, or a relative phase change in the modulated continuous RF signals over time is determined.

9. Method according to one of the preceding claims, wherein determining an image of the object undergoing examination (12) is additionally determined using the received MR signals.

10. Method according to one of the preceding claims, furthermore comprising:
- determining a frequency range of the received MR signals from the object undergoing examination (12); and
- emission of the continuous RF signals outside the determined frequency range of the MR signals.

11. Method according to one of the preceding claims, wherein the continuous RF signals are emitted by means of a plurality of RF coils (2).

12. Method according to claim 11, wherein the plurality of RF coils (2) emit simultaneously, and the continuous RF signals of the individual RF coils (2) differ in respect of one or more emission properties, comprising their frequency, their relative phase, their amplitude, their direction of electromagnetic polarisation, and their signal modulation.

13. Method according to claim 11 or 12, wherein the plurality of RF coils (2) emit sequentially.

14. Method according to claim 12 or 13, wherein the one or more emission properties vary sequentially.

15. Method according to one of claims 11 to 14, wherein at least one of the RF coils (2) is shifted in space.

16. Method according to one of the preceding claims, wherein the object undergoing examination (12) undergoes a dynamic change, and wherein only the variations in the continuous modulated RF signals are taken into account when an image of the object undergoing examination (2) is determined, on the basis of the dynamic change.

17. Method according to one of the preceding claims, wherein the amplitudes of the continuous RF signals are substantially the same size as the amplitudes of the MR signals.

18. MR system for performing electrical impedance tomography (EIT) on an object undergoing examination (12), wherein the MR system (1) has a plurality of RF coils (2), an MR control unit (13) and a memory unit (16), wherein the memory unit (16) stores control information that may be executed by the MR control unit (13), and wherein the MR system takes a form such that, when the control information is executed in the MR control unit (13), it performs the following steps:
- emitting an RF pulse by means of a first group of RF coils (2), for the purpose of exciting spin in the object undergoing examination (12);
- receiving MR signals from the object undergoing examination (12), based on the RF pulse, by means of a second group of RF coils;
- emitting continuous RF signals having amplitudes in the same order of magnitude as the amplitudes of the MR signals, by means of at least one of the RF coils (2);
- receiving modulated continuous RF signals from the object undergoing examination (12), in response to the continuous RF signals, by means of the second group of RF coils (2); and
- determining an image of the object undergoing examination (12) on the basis of the modulated continuous RF signals;
wherein emission of the continuous RF signals and receiving of the modulated continuous RF signals are performed simultaneously with the receiving of MR signals.

19. MR system for performing electrical impedance tomography (EIT) on an object undergoing examination (12), wherein the MR system comprises a plurality of RF coils (2), an MR control unit (13) and a memory unit (16), wherein the memory unit (16) stores control information that may be executed by the MR control unit (13), and wherein the MR system takes a form such that, when the control information is executed in the MR control unit (13), it performs the method from one of claims 2 to 17.

20. Computer program product, which comprises a program that is directly loadable into a memory of an MR control unit (13) of an MR system (1), having programming means to perform the steps of the method according to one of claims 1 to 17 when the program is executed in the MR control unit (13) of the MR system (1).

21. Electronically readable data medium having electronically readable control information stored thereon, wherein the control information is configured such that, when the data medium is used in an MR control unit (13) of an MR system (1), it performs the method according to one of claims 1 to 17.

## Revendications

1. Procédé pour effectuer une tomographie par impédance électrique (EIT) sur un objet (12) à examiner à l'aide d'une installation (1) RM, comprenant une pluralité de bobines (2) HF, ayant les stades suivants :
- émission d'une impulsion HF au moyen d'un premier groupe de bobines (2) HF pour exciter des spins dans l'objet (12) à examiner ;
- réception de signaux RM de l'objet (12) à examiner, reposant sur l'impulsion HF au moyen d'un deuxième groupe de bobines (2) HF ;
- émission de signaux HF continus, qui ont des amplitudes de l'ordre de grandeur des amplitudes des signaux RM, au moyen d'au moins une bobine (2) HF ;
- réception de signaux HF continus modulés de l'objet (12) à examiner, en réponse aux signaux HF continus, au moyen du deuxième groupe de bobines (2) HF ; et
- détermination d'une image de l'objet (12) à examiner, reposant sur les signaux HF continus modulés ;
dans lequel on effectue l'émission des signaux HF continus et la réception des signaux HF continus modulés simultanément à la réception des signaux RM.

2. Procédé suivant la revendication 1, dans lequel on choisit la fréquence ou les fréquences des signaux HF continus de manière à ne pas produire la condition de résonance pour l'excitation de spins nucléaires de l'objet (12) à examiner.

3. Procédé suivant l'une des revendications précédentes, dans lequel la fréquence ou les fréquences des signaux HF continus est à l'extérieur de la plage de fréquence des signaux RM de l'objet (12) à examiner et à l'intérieur d'une plage de réception du deuxième groupe de bobines (2) HF.

4. Procédé suivant l'une des revendications précédentes, dans lequel les signaux HF continus comprennent un signal HF de monofréquence d'amplitude constante.

5. Procédé suivant l'une des revendications précédentes, dans lequel la au moins une bobine (2) HF, qui est utilisée pour l'émission des signaux HF, est une bobine (2) HF d'émission d'un signal pilote-tone-navigator.

6. Procédé suivant l'une des revendications précédentes, dans lequel la au moins une bobine HF, qui est utilisée pour l'émission des signaux HF continus, est intégrée à l'une des bobines (2) HF du deuxième groupe de bobines (2) HF.

7. Procédé suivant l'une des revendications 1 à 5, dans lequel la au moins une bobine (2) HF, qui est utilisée pour l'émission des signaux HF continus, n'est pas intégrée à l'une des bobines (2) HF du deuxième groupe de bobines (2) HF et, par rapport à une bobine (2) HF du deuxième groupe de bobines (2) HF, est placée plus près de l'objet (12) à examiner.

8. Procédé suivant l'une des revendications précédentes, dans lequel la détermination d'une image de l'objet (12) à examiner comprend une détermination d'une phase des signaux HF continus modulés, dans lequel on détermine une phase absolue par rapport aux signaux HF émis ou une variation de phase relative en fonction du temps des signaux HF continus modulés.

9. Procédé suivant l'une des revendications précédentes, dans lequel on effectue la détermination d'une image de l'objet (12) à examiner, en outre en utilisant les signaux RM reçus.

10. Procédé suivant l'une des revendications précédentes, comprenant, en outre :
- la détermination d'une plage de fréquence des signaux RM reçus de l'objet (12) à examiner ; et
- l'émission des signaux HF continus à l'extérieur de la plage de fréquence déterminée des signaux RM.

11. Procédé suivant l'une des revendications précédentes, dans lequel on émet les signaux HF continus au moyen de plusieurs bobines (2) HF.

12. Procédé suivant la revendication 11, dans lequel les plusieurs bobines (2) HF émettent simultanément et les signaux HF continus des diverses bobines (2) HF se recoupent dans une ou plusieurs propriétés d'émission, comprenant leur fréquence, leur phase relative, leur amplitude, leur direction de polarisation électromagnétique et leur modulation de signal.

13. Procédé suivant la revendication 11 ou 12, dans lequel les plusieurs bobines (2) HF émettent séquentiellement.

14. Procédé suivant la revendication 12 ou 13, dans lequel la une ou les plusieurs propriétés d'émission varient séquentiellement.

15. Procédé suivant l'une des revendications 11 à 14, dans lequel on déplace dans l'espace au moins l'une des bobines (2) HF.

16. Procédé suivant l'une des revendications précédentes, dans lequel l'objet (12) à examiner a une variation dynamique, et dans lequel, lors de la détermination d'une image de l'objet (12) à examiner, on ne tient compte que des variations des signaux HF modulés continus reposant sur la variation dynamique.

17. Procédé suivant l'une des revendications précédentes, dans lequel les amplitudes des signaux HF continus sont sensiblement égales aux amplitudes des signaux RM.

18. Installation RM pour effectuer une tomographie par impédance électrique (EIT) sur un objet (12) à examiner, l'installation (1) RM ayant une pluralité de bobines (2) HF, une unité (13) de commande RM et une unité (16) de mémoire, l'unité (16) de mémoire mettant en mémoire des informations de commande pouvant être réalisées par l'unité (13) de commande RM et l'installation RM étant constituée de manière à exécuter, lors de l'exécution des informations de commande dans l'unité (13) de commande RM, les stades suivants :
- émission d'une impulsion HF au moyen d'un premier groupe de bobines (2) HF pour exciter des spins dans l'objet (12) à examiner ;
- réception de signaux RM de l'objet (12) à examiner, reposant sur l'impulsion HF au moyen d'un deuxième groupe de bobines (2) HF ;
- émission de signaux HF continus, qui ont des amplitudes de l'ordre de grandeur des amplitudes des signaux RM, au moyen d'au moins une bobine (2) HF ;
- réception de signaux HF continus modulés de l'objet (12) à examiner, en réponse aux signaux HF continus, au moyen du deuxième groupe de bobines (2) HF ; et
- détermination d'une image de l'objet (12) à examiner, reposant sur les signaux HF continus modulés ;
dans lequel on effectue l'émission des signaux HF continus et la réception des signaux HF continus modulés simultanément à la réception des signaux RM.

19. Installation RM pour effectuer une tomographie par impédance électrique (EIT) sur un objet (12) à examiner, dans laquelle l'installation RM a une pluralité de bobines (2) HF, une unité (13) de commande RM et une unité (16) de mémoire, l'unité (16) de mémoire mettant en mémoire des informations de commande pouvant être exécutées par l'unité (13) de commande RM, et l'installation RM étant constituée pour exécuter le procédé suivant l'une des revendications 2 à 17 lors de l'exécution des informations de commande dans l'unité (13) de commande RM.

20. Produit de programme d'ordinateur, qui comprend un programme pouvant être chargé directement dans une mémoire d'une unité (13) de commande RM d'une installation (1) RM, comprenant des moyens de programme pour effectuer les stades du procédé suivant l'une des revendications 1 à 17, lorsque le programme est réalisé dans l'unité (13) de commande RM de l'installation (1) RM.

21. Support de données déchiffrables électroniquement, sur lequel sont mis en mémoire des informations de commande déchiffrables électroniquement, qui sont conformées de manière à ce qu'elles effectuent, lors de l'utilisation du support de données dans une unité (13) de commande RM d'une installation (1) RM, le procédé suivant l'une des revendications 1 à 17.
